(19) Europäisches Patentamt
European Patent Office
Office européen des brevets

(11) EP 1 380 282 B1

(12) **EUROPEAN PATENT SPECIFICATION**

(45) Date of publication and mention of the grant of the patent:
**30.11.2005 Bulletin 2005/48**

(21) Application number: **02720512.9**

(22) Date of filing: **19.04.2002**

(51) Int Cl.[7]: **A61K 7/06**, A61K 7/08, A61K 7/11, A61K 7/075

(86) International application number:
**PCT/JP2002/003903**

(87) International publication number:
**WO 2002/085316 (31.10.2002 Gazette 2002/44)**

# (54) COSMETIC HAIR PREPARATION COMPOSITION

KOSMETISCHE HAARPRÄPARATZUSAMMENSETZUNG

COMPOSITION DE PREPARATION COSMETIQUE CAPILLAIRE

(84) Designated Contracting States:
**DE FR GB NL**

(30) Priority: **20.04.2001 JP 2001123314**

(43) Date of publication of application:
**14.01.2004 Bulletin 2004/03**

(73) Proprietor: **GE TOSHIBA SILICONES CO., LTD.
Tokyo 106-8550 (JP)**

(72) Inventors:
- **SUENAGA, Koji
c/o GE TOSHIBA SILICONES CO., LTD.
Minato-ku, Tokyo 106-8550 (JP)**
- **SATO, Akinori
c/o GE TOSHIBA SILICONES CO., LTD.
Minato-ku, Tokyo 106-8550 (JP)**

(74) Representative: **Granleese, Rhian Jane
Marks & Clerk
90 Long Acre
London WC2E 9RA (GB)**

(56) References cited:
**GB-A- 2 303 857**
**JP-A- 1 113 313**
**JP-A- 6 080 559**
**JP-A- 10 114 622**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 380 282 B1

## Description

### Technical Field

The present invention relates to hair cosmetics containing colloidal silica core/silicone shell particles and polyorganosiloxane, and more particularly, to a hair care cosmetic composition not only giving a smooth feeling but also has satisfactory setting retention.

### Background Art

A hair care cosmetic composition generally contains a high-molecular compound in order to impart hair setting retention. Specifically, a high-molecular compound such as a polyvinylpyrrolidone-based high-molecular compound, a polyvinylether-based high-molecular compound, a polyvinyl acetate-based high-molecular compound is used therein, or an acryl-based high-molecular compound. The blend of such a high-molecular compound, however, cannot be fully satisfactory in terms of smoothness and combing easiness even though it is capable of imparting high setting retention.
Meanwhile, various patent documents disclose that the use of silicone is advantageous in giving smoothness to hair. However, there exists a problem, that excessive use of silicone extremely softens hair to impair setting retention and, deprives hair, especially when they are thin, of firmness to make it difficult to handle.
Further, the specification of US Patent No. 4902499 discloses a hair care cosmetic composition comprising a specific silicone polymer dissolved in a volatile carrier in order to increase hair setting retention. This hair care cosmetic composition is prepared in such a manner that silicone gum, silica or silicone elastomer, and a silicone resin are dissolved in a volatile carrier such as oxtamethyltetrasiloxane, and the resultant composition is useful for hair cosmetics. Further, the specification of Japanese Patent No. 3043816 discloses a hair care cosmetic composition which gives setting retention and smoothness to hair by the use of cross-linked silicone. Further, Japanese Patent No. 10 114 622 discloses the use of silica core/silicone shell particles for hair cosmetics. It is noted that the invention disclosed in this laid-opened publication comprises silica core/silicone shell particles independently and in JP 10 114 622 any effect obtained when polyorganosiloxane is blended therein is not described.
As mentioned above, the use of specific silicone as hair care cosmetics has been already known, but any of hair care cosmetics obtained by these well-known arts is not fully satisfactory in terms of hair setting retention and feeling such as smoothness, and further improvement has been desired.
The present invention is made from such viewpoints. It is an object of the present invention to provide a hair care cosmetic composition which does not only give a smooth feeling but also has satisfactory setting retention.

### Disclosure of the Invention

As a result of assiduous studies in consideration of the above circumstances, the inventors of the present invention have found out that the blend of silica core/silicone shell particles, in which colloidal silica and polyorganosiloxane are siloxane-bonded together, and polyorganosiloxane in hair cosmetics at a specific weight ratio not only imparts satisfactory setting retention to the hair cosmetics, but also unexpectedly can give smoother feeling to hair than single use of polyorganosiloxane.
Specifically, a hair care cosmetic composition according to the present invention is characterized in that it contains (A) colloidal silica core/silicone shell particles consisting of (1) 90 wt% to 10 wt% cores of colloidal silica and (2) 10 wt% to 90 wt% shells of polyorganosiloxane represented by the mean formula $R^1{}_aSiO_{(4-a)/2}$ (where $R^1$s are the same or different and each is a substituted or unsubstituted monovalent hydrocarbon group and a is a number of 1.80 to 2.20), and (B) polyorganosilaxane, (A)/(B), namely, the weight ratio between the component (A) and the component (B) being 1/1000 to 4/1 and the sum of the component (A) and the component (B) being 0.1 wt% to 10 wt% of the total weight.
According to the present invention, the use of colloidal silica core/silicone shell particles and polyorganosiloxane together can not only give high setting retention to hair but also give more excellent hair care properties (smoothness and combing easiness) than those when polyorganosiloxane is used independently.

### Best Mode for Carrying out the Invention

Next, a preferred embodiment of the present invention will be explained.
A hair care cosmetic composition of an embodiment of the present invention contains (A) colloidal silica core/silicone shell particles consisting of (1) 90 wt% to 10 wt% cores of colloidal silica and (2) 10 to 90 wt% shells of polyorganosiloxane represented by the mean formula $R^1aSiO_{(4-a)/2}$ (where $R^1$s are the same or different and each is a substituted or unsubstituted monovalent hydrocarbon group and a is a number of 1.80 to 2.20), and (B) polyorganosiloxane, the weight ratio between the component (A) and the component (B) being 1/1000 to 4/1 and the sum of the component (A) and the component (B) being 0.1 to 10 wt% of the total weight.

Here, the colloidal silica core/silicone shell particles, which are those described in the specification of Japanese Patent No. 3 223 333, basically has such a form that colloidal silica serves as cores and at least a part of the surface thereof is covered with silicone as shells, but may take the form of including a small amount of separated silicon particles.

To explain in more detail, the colloidal silica core/silicone shell particles of the present invention may take the following three kinds of forms: (1) both ends of polyorganosiloxane are bonded with the surface of silica by siloxane bonding; (2) one end of polyorganosiloxane is bonded with the surface of silica by siloxane bonding and the other end is terminated with a hydroxyl group; and (3) both ends of polyorganosiloxane are terminated with hydroxyl groups and no siloxane bonding with the surface of silica is included.

The component (1) colloidal silica of the colloidal silica core/silicone shell particles is aqueous dispersed particles with $SiO_2$ being a basic unit and the average particle diameter thereof is 4 nm to 300 nm, more preferably, 30 nm to 150 nm.

Incidentally, there are acid colloidal silica and alkaline colloidal silica in terms of property classification, and either of them is usable by appropriate selection depending on conditions of emulsion polymerization in producing the core-shell particles. For example, the use of acid colloidal silica is preferable for emulsion polymerization under acid conditions using an anionic surfactant.

The weight ratio of the component (2) of the colloidal silica core/silicone shell particles, namely, the shells of polyorganosiloxane according to the present invention is selected within a range of 10 wt% to 90 wt%. The reason is that polyorganosiloxane, when it is less than 10 wt%, cannot fully cover the surface of colloidal silica, resulting in colloidal silica core/silicone shell particles inferior in stability, and polyorganosiloxane, when it is more than 90 wt%, does not produce a sufficient reinforcing property of colloidal silica, resulting in elastomeric cured material lacking mechanical properties. In short, any weight ratio falling outside the aforesaid range does not result in a hair care cosmetic composition which not only gives a smooth feeling but also has satisfactory setting retention.

In the aforesaid mean formula $R^1{}_aSiO_{(4-a)/2}$ representing the shells of polyorganosiloxane of the core/shell particles, $R^1$s bonded with silicon atoms are the same or different and each is a substituted or unsubstituted monovalent hydrocarbon group.

Specific examples of the unsubstituted monovalent hydrocarbon group are a straight-chain or branched-chain alkyl group such as methyl, ethyl, propyl, hexyl, octyl, decyl, hexadecyl, and octadecyl; an aryl group such as phenyl, naphthyl, and xenyl; an aralkyl group such as benzyl, β-phenylethyl, methylbenzyl, and a naphthylmethyl group; a cycloalkyl group such as cyclohexyl and cyclopenthyl; and so on.

Specific examples of the substituted monovalent hydrocarbon group are generally 3, 3, 3-trifluoropropyl, 3-f luoropropyl, and so on, each of which is a group in which a hydrogen atom of the aforesaid unsubstituted monovalent hydrocarbon group is substituted by a halogen atom such as fluorine and chlorine.

The methyl group is preferable as $R^1$ since it is easily obtained or useful.

The hair care cosmetic composition according to the present invention can be generally manufactured in the following procedure. Specifically, prepared are (1) colloidal silica (core component) and (2) organosiloxane which has a structural unit represented by $R^2{}_nSiO_{(4-n)/2}$ (where $R^2$s are the same or different and each is a substituted or unsubstituted monovalent hydrocarbon group and n is a number of 0 to 3), and which contains no hydroxyl group, the number of silicon atoms thereof being 2 to 10. Next, organosiloxane is condensation polymerized with colloidal silica in a water medium in the presence of a surfactant so that an emulsion of the colloidal silica core/silicone shell particles is prepared.

In preparing the above-mentioned emulsion of the colloidal silica core/silicone shell particles, the combination of acid colloidal silica and an anionic surfactant or the combination of alkaline colloidal silica and a cationic surfactant is selected in order to maintain colloidal silica in a stable condition . An amount of water in this emulsification is 43 to 90 parts by weight, preferably, 67 to 400 parts by weight relative to 100 parts by weight that is the total weight of the colloidal silica component and the organosiloxane component, and the concentration of emulsion solid content is 10 wt% to 70 wt%, preferably 20 wt% to 60 wt%. Further, the temperature in the process (condensation reaction and so on) of preparing the colloidal silica core/silicone shell particles is about 5°C to about 100°C. Incidentally, in the organosiloxane component constituting the silicone shells, a cross-linking agent such as silane having a functional group may be added in order to increase the strength of the shell.

**[0023]** - Since the emulsion of the colloidal silica core/silicone shell particles exhibits an acid property or an alkaline property, it is neutralized by the addition of alkali or acid in order to maintain long-term stability. Examples used here as an alkaline neutralizing agent are sodium hydroxide, thorium carbonate, thorium hydrogen carbonate, triethanolamine, and so on, and examples of an acid neutralizing agent are hydrochloric acid, sulfuric acid, nitric acid, acetic acid, oxalic acid, and so on.

The following are specific examples of the aforesaid polyorganosiloxane that is a source component of polyorganosiloxane forming the shells of the core/shell particles used in the present invention.

They are cyclic compounds such as hexamethylcyclotrisiloxane, oxtamethylcyclotetrasiloxane, decamethylcyclopentasiloxane, 1,3,5,7-tetramethyl-1,3,5,7-tetraphenylcyclotetrasiloxane, 1,3,5,7-tetrabenzyltetramethylcyclotetrasiloxane, and 1,3,5,7-tris-(3,3,3-trifluoroprophyl)trimethylcyclotetrasiloxane, and one of them or a mixture of two kinds or more thereof may be used.

As a silane compound used for introducing a group including an organic functional group, the following silanes are used.

Specific examples of the silane compound including the organic functional group are 3-aminopropylmethyldimethoxysilane,
3-aminopropyltrimethoxysilane,
N-(2-aminoethyl)-3-aminopropyltrimethoxysilane,
N-triethylenediaminepropylmethyldimethoxysilane,
3-glycidoxypropylmethyldimethoxysilane,
3,4-epoxycyclohexylethyltrimethoxysilane,
3-mercaptopropyltrimethoxysilane,
trifluoropropyltrimethoxysilane,
3-carboxypropylmethyldimethoxysilane, and so on, and one of them
or a mixture of two kinds or more thereof may be used.

An anionic surfactant or a cationic surfactant is appropriately used as the surfactant mainly playing an emulsifying function in preparing the emulsion of the colloidal silica core/silicone shell particles according to the present invention. As described above, the anionic surfactant is preferably used when the acid colloidal silica is used as a source while the cationic surfactant is preferably used when the alkaline colloidal silica is used as a source.

As the anionic surfactant used here, aliphatic substituted benzenesulfonic acid, aliphatic hydrogen sulfate, a mixture of unsaturated aliphatic sulfonic acid and hydroxylated aliphatic sulfonic acid are preferable, which are represented by the following general formulas respectively.

$$R^3C_6H_4SO_3H$$

$$R^3OSO_3H$$

$$R^4CH = CH(CH_2)_nSO_3H$$

$$R^4CH_2CH(OH)(CH_2)_nSO_3H$$

Note that in the formulas, $R^3$ is a monovalent aliphatic hydrocarbon group with the number of its carbon atoms being 6 to 30 (preferably 6 to 18), $R^4$ is a monovalent aliphatic hydrocarbon group with the number of its carbon atoms being 1 to 30 (preferably 6 to 18), and n is such an integer that the total number of carbon atoms becomes 6 to 30.

Specific examples of $R^3$ and $R^4$ are a hexyl group, an octyl group, a decyl group, a dodecyl group, a cetyl group, a stearyl group, a myricyl group, an oleyl group, a nonenyl group, an octynil group, a phytyl group, a pentadecadienyl group, and so on. Specific examples of the anionic surfactant having the $R^3$ group are hexylbenzene sulfonic acid, octylbenzene sulfonic acid, dodecylbenzene sulfonic acid, cetylbenzene sulfonic acid, octylsulfate, laurylsulfate, oleylsulfate, cetylsulfate, and so on. Examples of the anionic surfactant having the $R^4$ group are tetradecene sulfonic acid, hydroxytetradecane sulfonic acid, and so on.

Incidentally, anionic surfactant weak in catalysis, for example, sodium salt, ammonium salt, and triethanolamine salt among the anionic surfactants represented by the above general formulas are usable when they are used along with a polymerization catalyst, examples of such surfactants being sodium dodecylbenzenesulfonate, sodium octylbenzenesulfonate, ammonium dodecylbenzenesulfonate, sodium laurylsulfate, ammonium laurylsulfate, triethanolamine laurylsulfate, sodium tetradecenesulfonate, sodium hydroxytetradecenesulfonate, and so on.

The polymerization catalyst used together is generally a catalyst used in polymerization of low molecular weight organosiloxane, for example, aliphatic substituted benzenesulfonic acid, aliphatic hydrogen sulfate, a mixture of unsaturated aliphatic sulfonic acid and hydroxylated aliphatic sulfonic acid, hydrochloric acid, sulfuric acid, phosphoric acid, and so on, and even though these acid catalysts are preferable, they are not restrictive. In other words, any catalyst can be used together as long as it can cause polymerization of the low molecular weight organosiloxane.

The anionic surfactant is not limited to those represented by the aforesaid formulas. One kind or a mixture of two kinds or more is usable, for example, polyoxyethylene alkylether sulfuric ester or salt thereof such as polyoxyethylene (4) laurylethersulfate, polyoxyethylene(13)cetylethersulfate, polyoxyethylene(6)stearylethersulfate, polyoxyethylene(4) sodium laurylethersulfate, polyoxyethylene(4) ammonium octylphenylethersulfate; polyoxyethylene alkylether carboxylate ester or salt thereof such as polyoxyethylene(3)laurylethercarboxylate, polyoxyethylene(3)stearylethercarboxylate, polyoxyethylene(6) sodium laurylethercarboxylate, and polyoxyethylene(6) sodium octylethercalboxylate; and so on.

A usage amount of the anionic surfactant is 0.5 to 20 parts by weight (preferably 0.5 to 10 parts by weight) relative to the total amount 100 parts by weight of colloidal silica constituting the cores and organosiloxane constituting the shells. Inferior stability of the produced emulsion may possibly cause separation with the usage amount of less than 0.5 parts by weight, while the usage amount of more than 20 parts by weight may cause viscosity increase of the produced emulsion to lower its fluidity. Incidentally, in the case of using the polymerization catalyst together, the amount of the polymerization catalyst used together is preferably about 0.05 to about 10 parts by weight relative to the total 100 parts by weight of colloidal silica and organosiloxane.

Meanwhile, an example of the cationic surfactant is a quaternary ammonium surfactant represented by the following general formula (where, $R^5$ is aliphatic monovalent hydrocarbon group with the number of carbon atoms thereof being 6 or more, preferably 8 to 18, each of $R^6$, $R^7$, and $R^8$ is a monovalent organic group, and X is a hydroxyl group, a chlorine atom, or a bromine atom).

[Chemical Formula 1]

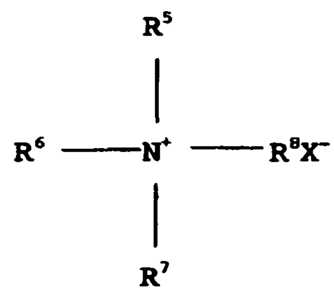

Specific examples of $R^5$ are hexyl, octyl, decyl, dodecyl, cetyl, stearyl, myricyl, oleyl, nonenyl, octynil, phytyl, pentadecadienyl, and so on. $R^6$, $R^7$, and $R^8$ are the same or different and each is a monovalent organic group, for example, an alkyl group such as methyl, ethyl, and propyl; and an alkenyl group such as vinyl and an allyl group; an aryl group such as phenyl, xenyl, and a naphthyl group; a cycloalkyl group such as cyclohexyl; and so on.

Specific examples of the aforesaid quaternary ammonium surfactant are lauryltrimethylammonium hydroxide, stearyltrimethylammonium hydroxide, dioctyldimethylammonium hydroxide, distearyldimethylammonium hydroxide, lauryltrimethylammonium chloride, stearyltrimethylammonium chloride, cetyltrimethylammonium chloride, dicocoyldimethylammonium chloride, distearyldimethylammonium chloride, benzalkonium chloride, stearyldimethylbenzylammonium chloride, and so on, and one or a mixture of two kinds or more can be used.

Note that, since the cationic surfactant is weak in catalysis, it is preferable to use together a polymerization catalyst, for example, alkali metal hydroxide such as lithium hydroxide, sodium hydroxide, potassium hydroxide, and rubidium hydroxide.

A usage amount of the cationic surfactant is 0.5 to 50 parts by weight (preferably 0.5 to 20 parts by weight) relative to the total amount 100 parts weight of colloidal silica constituting the cores and organosiloxane constituting the shells. With a usage amount less than 0.5 parts by weight, only insufficient cationic properties can be obtained and in addition, separation may possibly occur due to low stability of the produced emulsion. On the other hand, a usage amount more than 50 parts by weight causes viscosity increase of the produced emulsion to lower its fluidity. Incidentally, in the case of using the polymerization catalyst together, the amount of the polymerization catalyst used together is preferably 0 .05 to 10 parts by weight relative to the total amount 100 parts by weight of the colloidal silica and the organosiloxane.

Meanwhile, cationic colloidal silica core/silicone shell particles manufactured through the use of a method described in Japanese Patent Laid-open No. Hei 9-137062 can be used. Specifically, in this method emulsion polymerization is caused using an anionic surfactant and after the reaction is finished, a nonionic surfactant and/or an amphoteric surfactant are(is) added as a miscible surfactant, and a cationic surfactant is further added for conversion to cationic properties.

A silicone emulsion containing the colloidal silica core/silicone shell particles produced in such emulsion polymerization is a very stable emulsion, having an average particle diameter of 350 nm or smaller. It especially exhibits

good blend stability when being blended into water-based hair care cosmetics such as shampoo, hair rinse, hair conditioner, a hair treatment product, and a hair styling product.

As the component (B) polyorganosiloxane used along with the component (A) colloidal silica core/silicone shell particles, dimethyl silicone can be used which is represented by the following mean formula (where $R^1$s are the same or different, each being an (un) substituted monovalent hydrocarbon group, and a is a number of 1.80 to 2.20).

$$R^1{}_aSiO_{(4-a)/2}$$

The viscosity thereof is ranging from 5 mPa·s to 50, 000, 000 mPa·s, preferably, 20 mPa·s to 30, 000, 000 mPa·s. at 25°C. The viscosity less than 5 mPa·s results in only a small effect of giving smoothness to hair, and polyorganosiloxane with the viscosity more than 50,000, 000 mPa·s is practically difficult to manufacture. Silicone with a single viscosity may be used or silicones with different viscosities may be blended for use.

As amino-denatured silicone, (1) amino-functional polyorganosiloxane can be used whose amino content is about 0.02 to 3.0 milli-equivalent/g and includes the following units (a) and (b) :

(a) $R_aQ_bSiO_{(4-a-b)/2}$ unit
(b) $R_cSiO_{(4-c)/2}$ unit

[where a mole ratio between the $R_aQ_bSiO_{(4-a-b)/2}$ unit and the $R_cSiO_{(4-c)/2}$ unit is within the range of about 1:2 to about 1:65; a is a number within the range of 0 to 2; b is a number within the range of 1 to 3; a + b is 3 or less; c is a number within the range of 1 to 3; R is a monovalent hydrocarbon group or a substituted hydrocarbon group with the number of carbon atoms thereof being 1 to about 6; and Q is a polar group represented by the general formula $-R^1NHZ$ (where $R^1$ is a bivalent linkage group including a carbon and a hydrogen atom, including a carbon, a hydrogen, and an oxygen atom, or including a carbon, a hydrogen, and a sulfur atom; and Z is an atom or a group selected from a group consisting of an alkyl group and a $-CH_2CH_2NH_2$ group, the alkyl group including a hydrogen atom and 1 to 4 carbon atoms).

Specific examples of polyorganosiloxane used beside the above are methylphenyl silicone, polyether-denatured silicone, alkyl-denatured silicone, and so on, and one or a mixture of two kinds or more can be used. Dimethyl silicone and amino-denatured silicone are preferably used since they have a large effect of giving smoothness to hair. The polyorganosiloxane mentioned above can be manufactured by well known methods.

The polyorganosiloxane (B) may be in a silicone oil-in water emulsion or a silicone water-in-oil emulsion.

Direct blend of oily silicone is not problematic as a form of blending polyorganosiloxane in hair care cosmetics, but it is preferably blended in a form of emulsion in the case when it is blended in water-based hair care cosmetics such as shampoo, hair rinse, hair conditioner, a hair treatment product, and a hair styling product. Specifically, it is emulsified using water and various kinds of surfactants and blended while being dispersed in water. In this case, the obtained emulsion is classified by particle diameter into macroemulsion (dispersed particle diameter 400 nm or larger), a microemulsion (dispersed particle diameter 100 nm or smaller), and a miniemulsion with intermediate diameter (dispersed particle diameter 100 nm to 400 nm), all of which are usable without any limitation for usage.

However, the miniemulsion and the microemulsion are preferably added to shampoo in view of blend stability, and the macroemulsion, the miniemulsion, and the microemulsion are preferable for hair rinse, hair conditioner, a hair treatment product, and a hair styling product.

A well known method may be used as a method of producing the aforesaid silicone emulsion, and examples of such a method are emulsion polymerization which is carried out by using cyclic siloxane such as octamethyltetrasiloxane and dimethylsiloxane terminated with hydroxyl group as a monomer, an emulsifying method using an emulsifying machine such as a colloid mill, a line mill, a homomixer, and a homogenizer, an emulsifying method using an emulsifying machine in which an anchor mixer and a homomixer are integrated or an anchor mixer and a disper mixer are integrated, and other methods like these, but they are not restrictive.

As a surfactant used in preparing the silicone emulsion, all of the anionic, cationic, nonionic, and amphoteric surfactants are usable, and each may be used independently or two kinds or more of them may be used together.

Specific examples of the anionic surfactant are dodecylbenzenesulfonate, octylbenzenesulfonate, polyoxyethylene laurylsulfate, laurylsulfate, tetradecenesulfonate, and hydroxytetradecene sulfonate, and sodium salt, potassium salt, and triethanolamine salt thereof, and so on.

Examples of the cationic surfactant are lauryltrimethylammonium hydroxide, stearyltrimethylammonium hydroxide, dioctyldimethylammonium hydroxide, distearyldimethylammonium hydroxide, lauryltrimethylammonium chloride, stearyltrimethylammonium chloride, cetyltrimethylammonium chloride, dicocoyldimethylammonium chloride, distearyldimethylammonium chloride, benzalkonium chloride, stearyldimethylbenzylammonium chloride, and so on.

Examples of the nonionic surfactant are polyoxyethylenelaurylether, polyoxyethylene fatty acid ester, polyoxyethylene sorbitan fatty acid ester, sorbitan fatty acid ester, glycerol fatty acid ester, polyoxyethylene hydrogenated

caster oil, polyoxyethylene sorbitol fatty acid ester, and so on.

As the amphoteric surfactant, laurylamine oxide, lauryl betaine, cocoamidopropyl betaine, and so on can be used.

A weight ratio (A)/(B) between the component (A), namely, colloidal silica core/silicone shell particles and the component (B), namely, polyorganosiloxane which are used in the present invention is within the range of 1/1000 to 4/1. It is preferably 1/100 to 3/2, more preferably 1/20 to 1/1.

This is because, when the ratio (A)/(B) is less than 1/1000, blend of colloidal silica core/silicone shell particles does not bring about the advantageous effect of satisfactory setting retention and only gives a smoothness effect to the same extent as that brought about by single use of polyorganosiloxane. A blend ratio exceeding 4/1 brings about satisfactory setting retention but impairs smoothness of polyorganosiloxane.

The total usage amount of the component (A) colloidal silica core/silicone shell particles and the component (B) polyorganosiloxane which are used in the present invention is 0.1 wt% to 10 wt% of the total weight of a hair care cosmetic composition. It is preferably 0.5 wt% to 8 wt%, more preferably 1 wt% to 6 wt%. This is because a blend amount of less than 0.1 wt% does not bring about advantageous effects of setting retention and smoothness of hair while a blend amount exceeding 10 wt% causes deposition of silicone on hair more than necessary to give a sticky feeling to hair and in addition turns out be a cause of impairing setting retention.

In order to increase stability of the cosmetic preparation composition in preparing the hair care cosmetic composition according to the present invention, a nonionic surfactant may be added for use together before or after the emulsification of colloidal silica constituting the cores and organosiloxane constituting the shells. Note that, in the case of adding it before the emulsification, the catalysis of the aforesaid anionic surfactant or cationic surfactant may possibly be impaired, and therefore, when it is used, a weight ratio thereof is preferably in the range of 0 to 500 parts by weight relative to 100 parts by weight of the anionic surfactant or the cationic surfactant.

Here, specific examples of the nonionic surfactant are polyoxyethylene alkyl ether such as polyoxyethylene (6) laurylether, polyoxyethylene(7)cetylether, polyoxyethylene(20)stearylether, and polyoxyethylene(10)behenylether; polyoxyethylene alkylphenyl ether such as polyoxyethylene(3)octylphenylether and polyoxyethylene(18)nonylphenyl ether; polyethyleneglycol fatty acid ester such as polyethylene glycol monostearate (14E.0.) and polyethyleneglycol distearate (80E.0.); polyoxyethylene sorbitan fatty acid ester such as polyoxyethylene sorbitan monostearate (20E.0.), polyoxyethylene sorbitan monolaurate (6E.0.), polyoxyethylene sorbitan monopalmitate (20E.0.), polyoxyethylene sorbitan monostearate (6E.0.), and polyoxyethylene sorbitan trioleate (20E.0.); sorbitan fatty acid ester such as sorbitan monopalmitate and coconut oil fatty acid sorbitan; polyoxyethyleneglycerol fatty acid ester such as polyoxyethylene hydrogenated castor oil, polyoxyethylene sorbit tetraoleate, polyoxyethylene monooleate(15)glyceryl, and polyoxyethylene monostearate(15)glyceryl; polyoxyethylene polyoxypropylene alkyl ether such as polyoxyethylenephytosterol and polyoxyethylene(10)polyoxypropylene(4)cetylether; polyoxyethylene alkylamine such as polyoxyethylene(5)stearylamine; and polyoxyethylene alkylether phosphate such as polyoxyethylene(5) sodium cetylether phosphate.

Among these nonionic surfactants, those whose HLB is 6 to 20 is preferably used together since the resultant core/shell particles has a good emulsion stability.

The hair care cosmetic composition according to the present invention has as its essential components the core/shell particles and polyorganosiloxane, the shell particles consisting of colloidal silica serving as the cores and the shells of silicone covering the cores by way of silicon bonding. Accordingly, the action of the colloidal silica core/silicone shell particles gives satisfactory setting retention to hair, and in addition, the co-usage of colloidal silica core/silicone shell particles and polyorganosiloxane enables to give a smoother feeling than that brought about by the single use of polyorganosiloxane.

Specific examples of the hair care cosmetic composition according to the present invention are shampoo, hair rinse, hair conditioner, a hair treatment product, a hair styling product, hair mousse, hair cream, hair gel, and so on.

When the colloidal silica core/silicone shell particles of the present invention are used as a hair rinse agent, it is preferable to use one kind or two kinds or more of quaternary ammonium salt in the hair care cosmetic composition at a ratio of 0.1 wt% to 5 wt%. The ratio less than 0.1 wt% does not give a satisfactory rinse effect while the ratio more than 5 wt% results in high viscosity hair cosmetics, which is not suitable for use.

Examples of the quaternary ammonium salt are cetyltrimethylammonium chloride, stearyltrimethylammonium chloride, behenyltrimethylammonium chloride, behenyldimethylhydroxyethylammonium chloride, stearyldimethylammonium chloride, cetyltriethylammonium methylsulfate, and so on. Among them, stearytrimethylammonium chloride, behenyltrimethylammonium chloride, and stearyldimethylbenzylammonium chloride are especially preferable.

Meanwhile, when the colloidal silica core/silicone shell particles of the present invention are used for a washing agent such as shampoo, it is preferable that one kind or two kinds or more of the following is (are) used in hair cosmetic at a ratio of 5 wt% to 40 wt%: an anionic surfactant such as fatty acid soap, α-acyl sulfonate, alkyl sulfonate, alkylnaphthalene sulfonate, alkyl sulfate, polyoxyethylenealkylether sulfate, alkylamide sulfate, alkyl phosphate, alkylamide phosphate, alkyloylalkyltaurine salt, and N- acylamino acid salt; a nonionic surfactant such as glycerol fatty acid ester, for example, glycerol monostearate, glycerol monooleate, and so on, sorbitan fatty acid ester, for example, sorbitan

stearate, sorbitan oleate, and so on, polyoxyethylene sorbitan fatty acid ester, for example, polyoxyethylene coconut oil fatty acid sorbitan, polyoxyethylene sorbitan monopalmetate, polyoxyethylene sorbitan monostearate, and so on, polyoxyethylene alkylether, for example, polyoxyethylenelaurylether, polyoxyethylenestearylether, and so on, polyoxyethyleneglycol fatty acid ester, for example, polyoxyethyleneglycol monolaurate, polyethyleneglycol distearate, glycol distearate, and so on, and alkylalkanolamide, for example, diethanolamide laurate, coconut oil fatty acid diethanolamide, and so on; and an amphoteric surfactant such as betaine, for example, betaine lauryldimethylaminoacetate, betaine stearyldimethylaminoacetate, 2-alkyl-N-carboxymethyl-N-hydroxyethylimidazolinium betaine, coconut oil fatty acid amide propyl betaine, amidepropyl betaine laurate, and so on, aminocarboxylate, and imidazoline derivative. The ratio of these surfactants less than 5 wt% results in inferior washability and inferior foaming in washing, while the ratio more than 40 wt% results in high viscosity of the obtained hair cosmetic, which is not suitable for use.

It is permissible to blend the following in the hair care cosmetic composition of the present invention according to its intended use, that is, oil such as fluid paraffin, squalane, lanolin derivative, higher alcohol, and various kinds of ester oil; water-soluble oil such as ethyleneglycol, propyleneglycol, glycerol, and sorbitol polyethyleneglycol; moisturizer such as hyaluronic acid, chondoroitin acid, and pyrrolidone carboxylate; a thickener such as carboxy vinyl polymer; a cationic high polymer such as cation-denatured cellulose ether derivative, polyvinylpyrrolidone derivative quaternary ammonium, diallyl dimethylammonium chloride, polyamide derivative quaternary ammonium, polyoxyethylene polyalkylene, and polyamine; an ultraviolet absorbent; odor; and so on.

Next, specific examples of the present invention will be explained.

The terms, parts and % represent parts by weight and wt% respectively unless otherwise specified.

The average particle diameter of colloidal silica and colloidal silica core/silicone shell particles used as a source was measured with a laser particle diameter analyzing system LPA-3000S/3100 (manufactured by Ohtsuka Electronics Co., Ltd.) adopting dynamic light scattering.

A graft ratio and a graft efficiency were calculated in the following method, assuming that the colloidal silica core/silicone shell particles are graft polymers, in other words, the colloidal silica cores are trunk polymers and the silicone shells are branch polymers.

Specifically, a predetermined amount (Y) of colloidal silica core/silicone shell particles (dried substances), which were obtained as a result of five-hour pressure-reduced drying of an emulsion containing the colloidal silica core/silicone shell particles at 40°C/0.5 mmHg, was put into cyclohexane and set in a shaking machine to undergo 24 hour shaking. Free organosiloxane is dissolved in cyclohexane by this shaking and insoluble material is centrifuged for collection at a rotation speed of 2300 rpm for 30 minutes using a centrifugal separator. Next, a weight (M) of the insoluble material obtained as a result of one-hour drying at 120°C with a vacuum drier was measured and the graft ratio and the graft efficiency were calculated from the following equation respectively.

[Equation 1]

$$\text{graft ratio} = (M - Y \times \text{core ratio in core shell particles}) / (Y \times \text{core ratio in core shell particles}) \times 100$$

[Equation 2]

$$\text{graft efficiency} = (M - Y \times \text{core ratio in core shell particles}) / (Y - Y \times \text{core ratio in core shell particles}) \times 100$$

A ratio of the shell parts of the colloidal silica core/silicone shell particles was calculated in the following procedure . Note that a nonvolatile amount [%] is an average value of respective values of three samples each being 2 g after they were heated at 105°C for 3 hours.

(1) Calculation of an effective amount [parts] of colloidal silica

$$\text{A usage amount [parts] of colloidal silica dispersion} \times \text{colloidal silica active ingredient [\%]}$$

(2) Calculation of an amount of a theoretical nonvolatile amount [%]

{(Colloidal silica active ingredient [parts] + a usage amount [parts] of organosiloxane + an emulsifier amount [parts])/a total usage amount [parts] of source} × 100

(3) Calculation of an amount [parts] of unpolymerized organosiloxane

{A total usage amount [parts] of a source x (a theoretical nonvolatile amount [%] - a nonvolatile amount [%])} /100

(4) Calculation of an amount [parts] of polymerized organosiloxane

A usage amount [parts] of organosiloxane - an amount [parts] of unpolymerized organosiloxane

(5) Calculation of a polymerization ratio

An amount [parts] of polymerized organosiloxane/a usage amount [parts] of organosiloxane.

(6) Calculation of a shell part ratio

An amount [parts] of polymerized organosiloxane/(an amount [parts] of polymerized organosiloxane + colloidal silica active ingredient [parts]) x 100.

(Preparation of a colloidal silica core/silicone shell emulsion)

204 part octamethylcyclotetrasiloxane was added to a mixture of 500 part of SNOWTEX OL-40 (manufactured by Nissan Chemical Industries, Ltd., an average particle diameter 84 nm, $SiO_2$ 40.8%, $Na_2O$ 0.0049%, pH 2.3; abbreviated as silica-1) which is acid colloidal silica, 647. 8 part of ion-exchange water, and 8.2 part of n-dodecylbenzenesulfonate (manufactured by Nissan Chemical Industries, Ltd., soft straight alkylbenzene sulphonate 5S), and the resultant mixture was pre-stirred by a homomixer. Thereafter, it was passed through a homogenizer twice at a pressure of 300 $kgf/cm^2$ for emulsification and dispersion.

Next, the above emulsified dispersion was transferred to a separable flask equipped with a condenser, a nitrogen gas introducing port and an agitator, and after being heated at 85°C for 5 hours while being stirred and mixed, the mixture was maintained at 5°C for 48 hours to allow polymerization to occur. Next, an aqueous solution of sodium carbonate was added into the separable flask to neutralize the colloidal silica core/silicone shell emulsion to pH 7. Analysis of the obtained emulsion under the condition of 105°C in 3 hours indicated a nonvolatile content of 30.5% and a polymerization ratio of octamethyl cyclotetrasiloxane turned out to be 99.3%.

When particle diameter analysis was further conducted using a laser particle diameter analyzing system, complete disappearance of single dispersion particle diameter distribution of colloidal silica and new appearance of single dispersion particle diameter were observed, the former having its peak around 84 nm of the average particle diameter and the latter having its peak around 150 nm. Further, by the observation with an electron microscope, only a silicon particle figure was confirmed and no figure of colloidal silica particles was observed. This made it clear that

the aforesaid nonvolatile portion of the emulsion was colloidal silica core/silicone shell particles.

Meanwhile, a portion of the aforesaid colloidal silica core/silicone shell emulsion was put into a large amount of acetone to precipitate and collect by filtration the colloidal silica core/silicone shell particles, which were thereafter dried at 50°C for 12 hours with a vacuum drier, thereby obtaining aggregate of the colloidal silica core/silicone shell particles. Assuming that this aggregate was a graft polymer, the graft ratio and graft efficiency thereof were 41.7% respectively.

(Preparation of a silicone emulsion-1)

15 part of polyoxyethylene(2) sodium laurylethersulfate and 15 part of sodium laurylsulfate were uniformly dispersed in 468.5 part of ion-exchange water. 500 part of polydimethylsiloxane terminated with hydroxyl groups at both ends whose kinetic viscosity is 85 $mm^2/s$ at 25°C was added thereto, and after pre-mixing by stirring, processing with a pressurizing homogenizer (pressure 500 $kgf/cm^2$) was conducted three times, thereby obtaining an emulsion containing polydimethylsiloxane terminated with hydroxyl groups at both ends. 1.5 part of sulfuric acid was added to this emulsion, and 15-hour reaction was carried out at 15°C while stirring.

Next, a 10% sodium carbonate aqueous solution was added up to pH 7 and the polymerization reaction was stopped, thereby obtaining a silicone emulsion-1. When the average particle diameter of this emulsion was measured using the laser particle diameter analyzing system, it turned out to be 200 nm. Further, this emulsion was put into a large amount of isopropyl alcohol to precipitate polymers, and residual isopropyl alcohol and water were completely vaporized, thereby obtaining polyorganosiloxane. The kinetic viscosity of this polyorganosiloxane was 1,000,000 $mm^2/s$ at 25°C.

(Preparation of a silicone emulsion-2)

200 part of 2-aminoethyl-3-aminopropyl containing polydimethylsiloxane with its kinetic viscosity at 25°C being 1000 $mm^2/s$ and with its amino content being 0.6 milli-equivalent/g, 10 part of polyoxyethylene(9)laurylether, and 5 part of 70% aqueous solution of polyoxyethylene(40)octylphenylether were blended, thereby obtaining a pre-mixture. Next, 5.0 part of first portion of water was slowly added to this mixture. The obtained mixture was stirred for 15 minutes, thereby obtaining a uniform and satisfactory emulsion.

Subsequently, the remaining 776.5 part of water was added. Further, 3.5 part of acetic acid was added to this emulsion to adjust its pH to 7.5, thereby obtaining a silicone emulsion-2. The measurement with an N4 PLUS particle diameter measuring device manufactured by Coulter Counter showed that the average particle diameter of the obtained emulsion was 20 nm.

(Preparation of a silicone emulsion-3)

360 part of polydimethylsiloxane with its kinetic viscosity at 25°C being 200 $mm^2/s$ and 240 part of polyorganosiloxane with its viscosity at 25°C being 20, 000, 000 mPa · s were mixed to uniformity. 13.3 part of polyoxyethylene (4)laurylether, 10.9 part of polyoxyethylene(23)laurylether, and 60 part of water were added thereto and fully mixed, and thereafter the remaining water 315.8 part was added, thereby obtaining a silicone emulsion-3 which contains polyorganosiloxane. The measurement with an LS-230 manufactured by Coulter Counter showed that the average particle diameter thereof was 5 μm.

(Preparation of a silicone emulsion-4)

327.3 part of polydimethylsiloxane with its kinetic viscosity at 25°C being 200 $mm^2/s$, 218.2 part of polyorganosiloxane with its viscosity at 25°C being 20,000,000 mPa · s, and 54.5 part of 2-aminoethyl-3-aminopropyl containing polydimethylsiloxane with its kinetic viscosity at 25°C being 1000 $mm^2/s$ and with its amino content being 0.6 milli-equivalent were mixed up to uniformity. 13.3 part of polyoxyethylene (4) laurylether , 10.9 part of polyoxyethylene (23) laurylether, and 60 part of water were added thereto and fully mixed, and thereafter, the remaining 315.8 part of water was added, thereby obtaining a silicone emulsion-4 which contained polyorganosiloxane and amino-denatured silicone. The measurement with the LS-230 manufactured by Coulter Counter showed that the average particle diameter thereof was 3.5 μm.

Examples 1 to 5 and Comparative Examples 1 to 3 (Shampoo Compositions)

Shampoo compositions using the colloidal silica core/silicone shell emulsion (hereinafter, referred to as a core-shell emulsion), and the silicone emulsion-1 and silicone emulsion-2 which contained polyorganosiloxane were

prepared according to the formulation shown in Table 1.

The result of experiment evaluation of these shampoo compositions based on the evaluation method and evaluation criteria shown below will also be shown in Table 1.

[Table 1]

| Shampoo composition component | | E1 | E2 | E3 | E4 | E5 | CE1 | CE2 | CE3 |
|---|---|---|---|---|---|---|---|---|---|
| Silicone (A) | core/ shell emulsion | 0.03 | 1.3 | 0.7 | 2.7 | 1.3 | | | 6 |
| Silicone (B) | Silicone emulsion-1 | 3.98 | 3.2 | 1.6 | 6.4 | | | 4 | 0.4 |
| | Silicone emulsion-2 | | | | | 8 | | | |
| Polyoxyethylene (3) sodium laurylethersulfate | | 10 | 10 | 10 | 10 | 10 | 10 | 10 | 10 |
| Diethanolamide laurate | | 3.5 | 3.5 | 3.5 | 3.5 | 3.5 | 3.5 | 3.5 | 3.5 |
| Polyoxyethylene coconut oil-fatty acid monoethanolamide | | 0.2 | 0.2 | 0.2 | 0.2 | 0.2 | 0.2 | 0.2 | 0.2 |
| Glycerol | | 1 | 1 | 1 | 1 | 1 | 1 | 1 | 1 |
| Polyethyleneglycol distearate | | 3 | 3 | 3 | 3 | 3 | 3 | 3 | 3 |
| Polyoxyethylene hydrogenated castor oil | | 0.5 | 0.5 | 0.5 | 0.5 | 0.5 | 0.5 | 0.5 | 0.5 |
| Cationized cellulose | | 0.1 | 0.1 | 0.1 | 0.1 | 0.1 | 0.1 | 0.1 | 0.1 |
| Sodium chloride | | 0.75 | 0.75 | 0.75 | 0.75 | 0.75 | 0.75 | 0.75 | 0.75 |
| parabene | | 0.15 | 0.15 | 0.15 | 0.15 | 0.15 | 0.15 | 0.15 | 0.15 |
| Odor | | 0.2 | 0.2 | 0.2 | 0.2 | 0.2 | 0.2 | 0.2 | 0.2 |
| Purified water | | 76.59 | 76.1 | 78.3 | 71.5 | 71.3 | 80.6 | 76.6 | 74.2 |
| Silicone content % | | 2 | 2 | 1 | 4 | 2 | - | 2 | 2 |
| (A) / (B) | | 0.5/ 99.5 | 20/80 | 20/80 | 20/80 | 20/80 | - | 0/100 | 90/10 |
| Evaluated properties | Stability | ◎ | ◎ | ◎ | ◎ | ◎ | ◎ | ◎ | ◎ |
| | Smoothness (combing easiness) | 3.3 | 3.7 | 3.5 | 3.8 | 3.6 | 2.5 | 2.9 | 2 |
| E1= Example 1, E2= Example 2 , E3= Example 3 , E4= Example 4 , E5= Example 5 , CE1= Comparative Example 1, CE2= Comparative Example 2, CE3= Comparative Example 3 | | | | | | | | | |

The core/shell emulsion and silicone emulsions which were used have the following properties. Specifically, the core/shell emulsion has a D/Q ratio of 1/1, 30% silicone content, and the average particle diameter of 150 nm. The silicone emulsion-1 contains polydimethylsiloxane terminated with hydroxyl groups at both ends, its silicon content is 50%, the kinetic viscosity of base oil is one million $mm^2/s$, and its average particle diameter is 200 nm. The silicone emulsion-2 contains amino-functional silicone terminated with trimethylsilyl group at an end (2-aminoethyl-3-aminopropyl -containing polydimethylsiloxane, the amino content 0.6 milli-equivalent /g, the kinetic viscosity 1000 $mm^2/s$), its silicone content is 20%, and its average particle diameter is 20 nm.

Evaluation of "smoothness" was made for 12 panelists in such a manner that hair being 10 g in weight and 25 cm in length was washed with the shampoo compositions prepared as in Table 1, each being 1 g, for 1 minute, and after being rinsed with warm water at 40°C for 30 seconds, it was dried with a drier, and the results were represented

by points based on the following criteria. Note that each of the values shown in Table 1 is the average point of the 12 panelists.

5 very smooth
3 smooth
1 normal

"Stability" was evaluated based on the following criteria.

◎ stable for 6 weeks at a temperature of 50°C
○ stable for 4 weeks at a temperature of 50°C
× drainage and separation at a temperature of 50°C in 1 week

Examples 6 to 10 and Comparative Examples 4 to 6 (Hair Conditioner Compositions)

Hair conditioner compositions using the core/shell emulsion, and the silicone emulsion-3 and silicone emulsion-4 which contained polyorganosiloxane were prepared according to the formulation shown in Table 2.

The result of experiment evaluation of these hair conditioner compositions which were made based on the evaluation method and evaluation criteria described below will also be shown in Table 2.

[Table 2]

| Hair conditioner composition component | | E6 | E7 | E8 | E9 | E10 | CE4 | CE5 | CE6 |
|---|---|---|---|---|---|---|---|---|---|
| silicone (A) | core/ shell emulsion | 0.1 | 4 | 2 | 6 | 4 | | | 18 |
| Silicone (B) | Silicone emulsion-3 | 10 | 8 | 4 | 12 | | | 10 | 1 |
| | Silicone emulsion-4 | | | | | 8 | | | |
| Ethyleneglycol distearate | | 3 | 3 | 3 | 3 | 3 | 3 | 3 | 3 |
| Cetanol | | 2 | 2 | 2 | 2 | 2 | 2 | 2 | 2 |
| Propyleneglycol monostearate | | 3 | 3 | 3 | 3 | 3 | 3 | 3 | 3 |
| Glycerol monostearate | | 3.8 | 3.8 | 3.8 | 3.8 | 3.8 | 3.8 | 3.8 | 3.8 |
| Polyoxyethylene (3) stearate | | 3.8 | 3.8 | 3.8 | 3.8 | 3.8 | 3.8 | 3.8 | 3.8 |
| Cetyltrimethylammonium chloride | | 5 | 5 | 5 | 5 | 5 | 5 | 5 | 5 |
| Polyoxyethylene (20) cetylether | | 2 | 2 | 2 | 2 | 2 | 2 | 2 | 2 |
| 1,3-buthylene glycol | | 5 | 5 | 5 | 5 | 5 | 5 | 5 | 5 |
| Parabene | | 0.2 | 0.2 | 0.2 | 0.2 | 0.2 | 0.2 | 0.2 | 0.2 |
| Odor | | 0.2 | 0.2 | 0.2 | 0.2 | 0.2 | 0.2 | 0.2 | 0.2 |
| Purified water | | 61.9 | 60 | 66 | 54 | 60 | 72 | 62 | 53 |
| Silicone content % | | 6 | 6 | 3 | 9 | 6 | - | 6 | 6 |
| (A) / (B) | | 0.5/ 99.5 | 20/80 | 20/80 | 20/80 | 20/80 | - | 0/100 | 90/10 |

[Table 2] (continued)

| Hair conditioner composition component | | E6 | E7 | E8 | E9 | E10 | CE4 | CE5 | CE6 |
|---|---|---|---|---|---|---|---|---|---|
| Evaluated properties | Stability | ◎ | ◎ | ◎ | ◎ | ◎ | ◎ | ◎ | ◎ |
| | Smoothness (combing easiness) | 3.3 | 3.9 | 3.5 | 4 | 4.1 | 2 | 3 | 1.5 |
| E6= Example 6, E7= Example 7, E8= Example 8, E9= Example 9, E10= Example 10, CE4= Comparative Example 4, CE5= Comparative Example 5, CE6= Comparative Example 6 | | | | | | | | | |

The used core/shell emulsion has the same properties as those in the examples 1 to 5. The silicone emulsions have the following properties. Specifically, the silicone emulsion-3 has such base oil formulation, that is, polydimethylsiloxane (kinetic viscosity 200 $mm^2/s$) /polydimethylsiloxane (viscosity 20 million mPa·s)= 6/4, with a kinetic viscosity of base oil being 550 thousand $mm^2/s$, is nonionic, and has the silicone content of 60% and the average particle diameter of 5 μm, and the silicone emulsion-4 has such base oil formulation, that is, polydimethylsiloxane (kinetic viscosity 200 $mm^2/s$)/polydimethylsiloxane (viscosity 20 million mPa· s)/amino-functional siloxane = 6/4/1, the kinetic viscosity of base oil being 400 thousand $mm^2/s$, is nonionic, and has the silicone content of 60% and the average particle diameter of 3.5 μm. Amino-functional siloxane is amino-functional silicone terminated with trimethylsilyl group at an end (2-aminoethyl-3-aminopropyl containing polydimethylsiloxane), the amino content being 0.6 milli-equivalent/g, and the kinetic viscosity being 1000 $mm^2/s$.

Evaluation of "smoothness" was made for 12 panelists in such a manner that hair being 10 g in weight and 25 cm in length was washed with the hair conditioner compositions prepared as in Table 2, each being 1 g, for 1 minute, and after being rinsed with warm water at 40°C for 30 seconds, it was dried with a drier, and the results were represented by points based on the following criteria. Note that each of the values shown in Table 2 is the average point of the 12 panelists.

5 very smooth
3 smooth
1 normal

"Stability" was evaluated based on the following criteria.

◎ stable for 6 weeks at a temperature of 50°C
○ stable for 4 weeks at a temperature of 50°C
× drainage and separation at a temperature of 50°C

in 1 week

Examples 11 to 15 and Comparative Examples 7 to 9 (Hair Blow Product Compositions)

Hair blow product compositions using the core/shell emulsion, and the silicone emulsion-1 and silicone emulsion-2 which contained polyorganosiloxane were prepared according to the formulation shown in Table 3.

The result of experiment evaluation of these hair blow product compositions which were made based on the following evaluation method and evaluation criteria will also be shown in Table 3.

[Table 3]

| Hair blow product composition component | | E11 | E12 | E13 | E14 | E15 | CE7 | CE8 | CE9 |
|---|---|---|---|---|---|---|---|---|---|
| Silicone (A) | core/ shell emulsion | 0.03 | 1.3 | 0.7 | 2 | 1.3 | | | 6 |
| Silicone (B) | Silicone emulsion-1 | 4 | 3.2 | 1.6 | 4.8 | | | 4 | 0.4 |
| | Silicone emulsion-2 | | | | | 8 | | | |

[Table 3] (continued)

| Hair blow product composition component | | E11 | E12 | E13 | E14 | E15 | CE7 | CE8 | CE9 |
|---|---|---|---|---|---|---|---|---|---|
| Glycerol | | 2 | 2 | 2 | 2 | 2 | 2 | 2 | 2 |
| Hydrolitic collagen | | 1 | 1 | 1 | 1 | 1 | 1 | 1 | 1 |
| Stearyltrimethyl-ammonium chloride | | 2 | 2 | 2 | 2 | 2 | 2 | 2 | 2 |
| Ethanol | | 5 | 5 | 5 | 5 | 5 | 5 | 5 | 5 |
| Parabene | | 0.2 | 0.2 | 0.2 | 0.2 | 0.2 | 0.2 | 0.2 | 0.2 |
| Odor | | 0.2 | 0.2 | 0.2 | 0.2 | 0.2 | 0.2 | 0.2 | 0.2 |
| Purified water | | 85.6 | 85.1 | 87.3 | 82.8 | 80.3 | 89.6 | 85.6 | 83.2 |
| Silicone content % | | 2 | 2 | 1 | 3 | 2 | - | 2 | 2 |
| (A) / (B) | | 0.5/ 99.5 | 20/80 | 20/80 | 20/80 | 20/80 | - | 0/100 | 90/10 |
| Evaluated properties | Stability | ◎ | ◎ | ◎ | ◎ | ◎ | ◎ | ◎ | ◎ |
| | Smoothness (combing easiness) | 3.2 | 4 | 3.5 | 4.1 | 3.7 | 2 | 2.5 | 1.4 |
| | Hair setting retention | 3 | 5 | 3 | 5 | 5 | 1 | 1 | 5 |

E11= Example 11, E12= Example 12, E13= Example 13, E14= Example 14, E15= Example 15, CE7= Comparative Example 7, CE8= Comparative Examples 8, CE9= Comparative Example 9

Note that all of the used core/shell emulsion, silicone emulsion-1, and silicone emulsion-2 have the same properties as those in the examples 1 to 5.

Evaluation of "smoothness" was made for 12 panelists in such a manner that hair being 10 g in weight and 25 cm in length was washed with the hair blow compositions prepared as in Table 3, each being 1 g, for 1 minute, and after being rinsed with warm water at 40°C for 30 seconds, it was dried with a drier, and the results were represented by points based on the following criteria. Note that each of the values shown in Table 3 is the average point of the 12 panelists.

5 very smooth
3 smooth
1 normal

The evaluation of "hair setting retention" was made in such a manner that hair being 10 g in weight and 25 cm in length was given uniform spray of each of the hair blow product compositions, each being 1g, which were prepared according to Table 3, and thereafter, this hair was wound around a curler being 1.2 cm in outer diameter, and dried with warm air at 40°C for 60 minutes. Subsequently, the hair was removed from the curler, a length L1 thereof immediately after it was hung vertically in an atmosphere at a temperature of 30°C and a relative humidity of 80% and a length L2 after it was left therein for 1 hour were measured, and curl retention was calculated based on the following formula.

$$\text{curl retention (\%)} = (25 - L2)/(25 - L1) \times 100$$

The evaluation of the hair setting retention was made based on the following criteria.

5 curl retention is 60% or more
3 curl retention is 30% or more and less than 60%
1 curl retention is less than 30%

"Stability" evaluation was made based on the following criteria.

◎ stable for 6 weeks at a temperature of 50°C
○ stable for 4 weeks at a temperature of 50°C
× drainage and separation at a temperature of 50°C in 1 week

Industrial Applicability

As explained hitherto, according to the present invention, colloidal silica core/silicone shell particles and polyorganosiloxane are used together, which allows hair to have not only high setting retention but also more excellent hair care properties (smoothness and combing easiness) than those given by the single use of polyorganosiloxane.

## Claims

**1.** A hair care cosmetic composition, comprising:

(A) colloidal silica core/silicone shell particles consisting of (1) 90 wt% to 10 wt% cores of colloidal silica and (2) 10 wt% to 90 wt% shells of polyorganosiloxane represented by a mean formula

$$R^1{}_aSiO_{(4-a)/2}$$

(wherein $R^1$s are the same or different and each is a substituted or unsubstituted monovalent hydrocarbon group and a is a number of 1.80 to 2.20); and

(B) a polyorganosiloxane,

wherein (A)/(B), namely, a weight ratio between the component (A) and the component (B) is 1/1000 to 4/1 and the sum of the component (A) and the component (B) is 0.1 wt% to 10 wt% of the total weight.

**2.** The hair care cosmetic composition as set forth in claim 1, wherein said (A) colloidal silica core/silicone shell particles are in a silicone emulsion obtained by emulsion polymerization, and an average particle diameter thereof is 350 nm or smaller.

**3.** The hair care cosmetic composition as set forth in claim 1 or 2, wherein said (A) colloidal silica core/silicone shell particles are in a silicone emulsion obtained by anionic or cationic emulsion polymerization.

**4.** The hair care cosmetic composition as set forth in any one of claims 1 to 3, wherein said (B) polyorganosiloxane has a viscosity of 5 mPa · s to 50,000,000 mPa · s at 25°C.

**5.** The hair care cosmetic composition as set forth in any one of claims 1 to 4, wherein said (B) polyorganosiloxane is dimethylsilicone and/or amino-denatured silicone.

**6.** The hair care cosmetic composition as set forth in any one of claims 1 to 5, wherein said (B) polyorganosiloxane is in a silicone oil-in-water emulsion or a silicone water-in-oil emulsion.

## Patentansprüche

**1.** Kosmetische Haarpflegemittelzusammensetzung, aufweisend:

(A) kolloidale Siliciumdioxidkern/Siliconmantel-Partikel, bestehend aus: (1) 90% bis 10 Gew.% Kernen aus kollodialem Siliciumdioxid und (2) 10% bis 90 Gew.% Mantel aus Polyorganosiloxan, dargestellt durch die allgemeine Formel:

$$R^1_aSiO_{(4-a)/2}$$

(worin die $R^1$ gleich oder verschieden sind und jedes eine substituierte oder unsubstituierte einwertige Kohlenwasserstoff-Gruppe ist und *a* eine Zahl von 1,80 bis 2,20 ist); und

(B) ein Polyorganosiloxan,

worin (A)/(B), d.h. das Gewichtsverhältnis zwischen der Komponente (A) und der Komponente (B), 1/1000 bis 4/1 beträgt und die Summe der Komponente (A) und der Komponente (B) 0,1% bis 10 Gew.% des Gesamtgewichts beträgt.

**2.** Kosmetische Haarpflegemittelzusammensetzung nach Anspruch 1, worin die (A) kolloidalen Siliciumdioxidkern/Siliconmantel-Partikel sich in einer Silicon-Emulsion befinden, die durch Emulsionspolymerisation erhalten wird, und der mittlere Partikeldurchmesser davon 350 nm oder weniger beträgt.

**3.** Kosmetische Haarpflegemittelzusammensetzung nach Anspruch 1 oder 2, worin die (A) kolloidalen Siliciumdioxidkern/Siliconmantel-Partikel sich in einer Silicon-Emulsion befinden, die durch anionische oder kationische Emulsionspolymerisation erhalten wird.

**4.** Kosmetische Haarpflegemittelzusammensetzung nach einem der Ansprüche 1 bis 3, worin das (B) Polyorganosiloxan bei 25°C eine Viskosität von 5 mPa·s bis 50.000.000 mPa·s hat.

**5.** Kosmetische Haarpflegemittelzusammensetzung nach einem der Ansprüche 1 bis 4, worin das (B) Polyorganosiloxan Dimethylsilicon und/oder Amino-denaturiertes Silicon ist.

**6.** Kosmetische Haarpflegemittelzusammensetzung nach einem der Ansprüche 1 bis 5, worin sich das (B) Polyorganosiloxan in einer Öl-in-Wasser-Emulsion von Silicon oder einer Wasser-in-Öl-Emulsion von Silicon befindet.

## Revendications

**1.** Composition cosmétique de soin capillaire, comprenant:

(A) des particules à noyau en silice colloïdale/coque en silicone constituées de (1) 90% en poids à 10% en poids de noyaux en silice colloïdale et (2) 10% en poids à 90% en poids de coques en polyorganosiloxane représenté par une formule moyenne

$$R^1_a SiO_{(4-a)/2}$$

(dans laquelle les radicaux $R^1$ sont identiques ou différents et chacun représente un groupe hydrocarbure monovalent substitué ou non substitué et *a* représente un nombre ayant une valeur de 1,80 à 2,20); et

(B) un polyorganosiloxane,
dans laquelle (A)/(B), à savoir, un rapport en poids situé entre le composant (A) et le composant (B) a la valeur de 1/1000 à 4/1 et la somme du composant (A) et du composant (B) est de 0,1% en poids à 10% en poids du poids total.

**2.** Composition cosmétique de soin capillaire tel qu'exposée dans la revendication 1,
dans laquelle lesdites (A) particules à noyau en silice colloïdale/coque en silicone se trouvent dans une émulsion de silicone obtenue par polymérisation en émulsion, et un diamètre moyen de particule de celles-ci est de 350 nm ou inférieur.

**3.** Composition cosmétique de soin capillaire tel qu'exposée dans la revendication 1 ou 2, dans laquelle lesdites (A) particules à noyau en silice colloïdale/coque en silicone se trouvent dans une émulsion de silicone obtenue par polymérisation en émulsion anionique ou cationique.

**4.** Composition cosmétique de soin capillaire tel qu'exposée dans l'une quelconque des revendications 1 à 3, dans laquelle ledit (B) polyorganosiloxane a une viscosité de 5 mPa·s à 50000000 mPa·s à 25°C.

**5.** Composition cosmétique de soin capillaire tel qu'exposée dans l'une quelconque des revendications 1 à 4, dans laquelle ledit (B) polyorganosiloxane est de la diméthylsilicone et/ou de la silicone amino-dénaturée.

**6.** Composition cosmétique de soin capillaire tel qu'exposée dans l'une quelconque des revendications 1 à 5, dans laquelle ledit (B) polyorganosiloxane se trouve dans une émulsion de silicone "huile dans eau" ou une émulsion de silicone "eau dans huile".